Europäisches Patentamt

European Patent Office

Office européen des brevets

(11) Publication number: **0 110 443**
**B1**

# EUROPEAN PATENT SPECIFICATION

(45) Date of publication of patent specification: 09.04.86

(51) Int. Cl.⁴: **C 07 C 2/14, C 07 C 2/20**

(21) Application number: 83201513.5

(22) Date of filing: 20.10.83

(54) **Preparation of propylene and butylene dimers.**

(30) Priority: 19.11.82 US 442999

(43) Date of publication of application:
13.06.84 Bulletin 84/24

(45) Publication of the grant of the patent:
09.04.86 Bulletin 86/15

(84) Designated Contracting States:
BE DE FR GB IT NL

(56) References cited:
US-A-2 414 380
US-A-3 194 800

JOURNAL OF THE AMERICAN CHEMICAL
SOCIETY, vol. 88, 5th September 1966, Easton
(US), YOSHINOBU ODAIRA et al.: "The
synthesis of olefinic cyanides from olefins by
means of palladium (II) cyanide", pages 4105-
4106

(73) Proprietor: SHELL INTERNATIONALE
RESEARCH MAATSCHAPPIJ B.V.
Carel van Bylandtlaan 30
NL-2596 HR Den Haag (NL)

(72) Inventor: Smutny, Edgar Josef
1701 Maryland
Houston Texas 77006 (US)

(74) Representative: Aalbers, Onno et al
P.O. Box 302
NL-2501 CH The Hague (NL)

Courier Press, Leamington Spa, England.

## Description

The present invention relates to a process for the selective preparation of dimers of propylene and/or n-butylenes by contracting an olefin reactant selected from the group consisting of propylene, 1-butene, 2-butene, and mixtures thereof, under mechanical agitation with a heterogeneous palladium cyanide catalyst at a temperature in the range from 50°C to 150°C, the quantity of said palladium cyanide being between 0.01 and 10% w, calculated on the weight of olefin reactant.

The mono-olefinic dimers of propylene and the n-butylenes are useful as intermediates in the synthesis of lubricants, plasticisers and surface active agents, particularly alcohols and alkylates. In the past, commercial production of mono-olefins in the $C_6$ to $C_8$ range has largely been achieved by catalytic oligomerization of ethylene. Economic incentive has developed, however, for the use of other feedstocks, particularly propylene and n-butylenes.

Under common conventional practice, propylene and butylene have been converted to dimers by reaction in the presence of a strong acid, particularly sulphuric acid, phosphoric acid, or boron trifluoride. The products of such conversions have substantial branching in the dimer molecules. An object of the present invention is a process which is selective in the production of dimers having limited branching in the molecular structure.

Olefins dimerization reactions using palladium cyanide are also known, but the results obtained in these prior described conversions are not satisfactory. For instance, US—PS 3,535,302 discloses that ethylene can be dimerized in such manner, but also indicates that when propylene was used as starting material only a polymeric product was obtained, whereas no reaction was observed for butylenes. Moreover, it has been described in J. Am. Chem. Soc. *88* (1966), page 4106, that the reaction of propylene in the presence of palladium cyanide and a polar solvent yields hydrogen cyanide, methacrylonitrile, 3-butenenitrile, crotononitrile, isobutyronitrile, and butyronitrile. In non-polar solvents, palladium cyanide was said to lead almost exclusively to the conversion of propylene to high polymers.

According US—PS 3,194,800 alpha-olefins can be polymerized to solid, high molecular weight polymers by contacting the alpha-olefins under agitation at a temperature between 50°C and 250°C with a heterogeneous palladium cyanide catalyst, using a weight ratio of alpha-olefin to cyanide salt of up to 10,000. As alpha-olefins suitable for this known process ethylene, propylene and 1-butene are mentioned. All examples of this US—PS are limited to the use of ethylene as a starting material and show that high molecular weight polyethylene is produced.

The Applicants have tested the latter process using propylene and n-butylenes as starting material and have discovered that indeed polymers, but predominantly dimers were produced with good selectivity. Moreover, the dimers have limited branching in the molecule.

It has now been found that process conversion and/or selectivity can alternatively be improved by the presence in the catalyst and reactant mixture of a relatively small quantity of one or more acids.

Thus, the invention provides a process for the selective preparation of dimers of propylene and/or n-butylenes by contacting an olefin reactant selected from the group consisting of propylene, 1-butene, 2-butene, and mixtures thereof, under mechanical agitation with a heterogeneous palladium cyanide catalyst at a temperature in the range from 50°C to 150°C, the quantity of said palladium cyanide being between 0.01 and 10% w calculated on the weight of olefin reactant, characterized in that the contact between the olefin reactant and the catalyst is carried out in the presence of an acid as a reaction accelerator and that said dimers are recovered from the reaction produce solution thus obtained.

In this definition of the present process and hereinafter the terms dimers is used herein to describe the $C_6$ addition product of two propylene molecules, the $C_8$ addition product of two butylene molecules, or the $C_7$ addition product of propylene and butylene.

As indicated above, mechanical agitation of the reaction mixture and limitation upon the quantity of palladium cyanide used as catalyst have both been found necessary to accomplish conversion to dimers rather than to a polymeric product. The product dimers are in large part of straight-chain structure. With propylene as reactant, for instance, more than 70% of the product dimers are n-hexenes in most cases.

A further necessary element of the invention is the presence of a heterogeneous palladium cyanide catalyst. The good results obtained by using this particular catalyst in the present process are remarkable because other transition metal cyanides, e.g., platinum cyanide and nickel cyanide, have been found to be ineffective under the reaction conditions used in the present process.

It is preferred to use palladium cyanide which has been ground into a fine particulate to expose a relatively high surface area. The palladium cyanide may be applied alone or may suitably be supported on or otherwise combined with inert solid carriers. Particularly preferred are catalysts prepared by grinding or ball milling the palladium cyanide together with one or more essentially insert solids such as carbon, silica, and the cyanides of platinum, nickel, zinc, and copper. As a rule, such grinding of the catalyst, either alone or together with such supporting materials, increases its activity and/or selectivity to dimers in the process. Activity may also be enhanced if the catalyst is thoroughly (e.g. for several hours) washed with water or an aqueous acid solution before it is contacted with the olefin reactant.

For carrying out the process of the invention, the olefin reactant and the palladium cyanide are charged to a suitable pressure reactor equipped for mechanical agitation of the solid catalyst and the liquid reactant in the absence of a substance capable of converting the palladium cyanide into a form which is soluble in the reaction mixture, which would be inconsistent with the intended application of a heterogeneous catalyst. Non-polar reaction solvents (other than substances capable of solubilizing the palladium cyanide) may be added to the reaction mixture if desired. In some cases the presence of certain organic solvents, such as toluene, mesitylene, nitrobenzene and benzonitrile has been found to enhance process conversion and/or selectivity. However, the use of a large quantity of such a reaction solvent often proves to be of disadvantage when consideration is given to its recovery from the product mixture, and the process of the invention is preferably carried out in the absence of any substantial quantity of such solvents. Most preferably, the reaction mixture is essentially free of added reaction solvents.

Examples of very suitable acids that may be used as accelerators are halogen acids, sulphuric acid, phosphoric acid, acetic acid, formic acid, trifluoroacetic acid and trifluorosulphuric acid. Hydrofluoric acid is particularly preferred. The quantity of such accelerator is not critical, and as little as 1% w calculated on palladium cyanide may be effective. Preferably, when acid is applied as the accelerator it is used in a quantity between 5 and 100% w, based on palladium cyanide. A larger quantity, for example, up to about ten times by weight that of palladium cyanide, may be used, however, and is preferred when the added accelerator is water.

Particular equipment employed for mechanical agitation of the reaction and catalyst is not critical to the invention; suitable mixing means include driven impellers, fluid jets, flow baffles.

As already indicated above, the use of a limited quantity of palladium cyanide relative to the olefin reactant with which it is contacted is an essential feature of the process according to the invention. Excessive quantities of catalyst promote formation of products other than dimers, particularly the production of polymers as suggested in the prior art. A quantity of palladium cyanide that is at least 0.01% w calculated on olefin reactant is effective. Preferably, the quantity of palladium cyanide is between 0.1 and 6% w, calculated on this same basis, an amount between 0.5 and 4% w being most preferred. Good results have been obtained using a quantity of catalyst that is between 1 and 3% w, calculated on olefin reactant.

The temperature of the catalyst and olefin reactant mixture during the dimerization reaction in the present process is preferably between 70 and 130°C, more preferably between 80 and 120°C, and most preferably about 100°C. The pressure is not critical. The reaction is very suitably carried out at the pressure of the liquid olefin reactant and product (or reactant, product and solvent) mixture at the operating temperature but may optionally be increased above this equilibrium level, for instance, by introduction of an inert gas, although this has not been found to benefit either conversion or selectively. Similarly, the reaction time is not critical to the invention. In general the contact with the catalyst is between 1 and 50 h for a propylene reactant and between 4 and 100 h for a butylene reactant.

As mentioned above, the process of the invention is in distinction to conventional dimerization processes selective to the production of dimer having limited branching in the molecule. The dimer products are comprised in very substantial proportion of straight chain (linear) alkenes and of alkenes having a single methyl substituent at an internal carbon atom. In the case of propylene dimers, about 50 to 95 per cent are linear hexenes, with the remainder almost entirely 2-methylpentenes. For butylene dimers, about 30 to 40 per cent are linear. Limited branching in the product molecule is particularly important when the dimers are utilized as intermediates in the synthesis of detergent chemicals, for which it is recognized that highly branched carbon chains are undesirable from the standpoint of biodegradation.

Following the contact between the olefin reactant and the palladium cyanide catalyst, the dimer is separated from the reaction mixture, which also contains lesser amounts of trimer, higher oligomers and other by-products in solution and solid catalyst and precipitated polymer. In a preferred embodiment of the present process, unreacted propylene and/or butylene are removed by evaporation and solid catalyst and polymer are removed by filtration, leaving a solution from which dimer is then separated by fractionation.

The invention is further illustrated by the following examples. Selectivities mentioned therein are calculated on the basis of the total quantity of dimer, trimer, oligomer, and polymer recovered from the product and isolated. No significant amount of any other process product was observed.

Comparative Example A

To a 80 ml autoclave reactor at dry ice temperature were added 3.1 mm of commercial palladium cyanide and 667 mm of 1-butene reactant. The mixture was heated to 100°C and maintained at that temperature with stirring for 40 h. A maximum pressure of 2.0 MPa was recorded. Conversion of the 1-butene was 3% to a product mixture containing between 40 and 50% w dimer. After hydrogenation, the dimer product was analyzed as being 35—40% n-octane and 60—65% 3-methylheptane.

Comparative Experiment B

To a stirred 80 ml autoclave reactor at dry ice temperature were added 713 millimoles (abbreviated hereinafter as mm) of propylene and 3.1 mm of commercial palladium cyanide power. The mixture was heated to 100°C and maintained at that temperature for 2 h. A maximum pressure of about 5.8 MPa was recorded. Of the propylene, 6% was converted to a product of the following distribution: 60% w dimers, 7% w trimers, 8% w higher oligomers (present in solution in the liquid reaction mixture), and 25% w polymer (i.e., as precipitate from the liquid reaction mixture).

### Comparative Experiment C

Commercial palladium cyanide was ground and sieved to particles having a diameter between 15 and 42 μm. The reactor was charged with 3.1 mm of these particles and the procedure of Comparative Experiment B was again followed. After a 2 h reaction at 100°C, 9% of propylene reactant was converted to a product having the following distribution: 64% w dimers, 12% w trimers, 7% w higher oligomers, and 17% w polymer. After a 16 h reaction, conversion was 19% to a product mixture containing 59% w dimers, 15% w trimers, 8% w higher oligomers, and 18% w polymers.

### Comparative Experiment D

The general procedures of Comparative Experiment B were again followed, using 6.3 mm commercial palladium cyanide as catalyst and 708 mm propylene reactant. A 2 h reaction at 100°C (5.5 MPa maximum pressure) converted 19% of the propylene to a mixture of 66% w dimers, 11% w trimers, 8% w higher oligomers and 15% w polymers. In a 16 h reaction under comparable conditions, conversion reached 31% and the product distribution was 56% w dimers, 11% w trimers, 9% w higher oligomers, and 24% w polymers.

### Comparative Experiment E

The procedures of Comparative Experiment B were again followed using as catalyst a ground commercial palladium cyanide. An amount of 30 ml of mesitylene reaction solvent was also added to the autoclave. A 2 h reaction at 100°C (maximum pressure 5.3 MPa) resulted in a conversion of 10% and a product distribution of 69% w dimers, 14% trimers, 8% w higher oligomers, and 9% w polymers.

When only 368 mm of propylene was charged together with the 3.1 mm of ground catalyst and the 30 ml solvent, the 2 h reaction resulted in an increase in conversion to 22%, but in a decrease in product dimers selectively to 53% w.

### Comparative Experiment F

A representative sample of the dimer product prepared in accordance with the procedures of Comparative Experiments B—E was analyzed by NMR. Of the product dimer molecules, 73% were found to have a straight chain structure, while the remaining 27% consisted essentially of 2-methylpentenes. The results of an NMR analysis of the dimers are presented in Table I.

### TABLE I

| Dimer structure | | Percent of total dimers |
|---|---|---|
| Straight chain dimer | | |
| 1-hexene | | 4% |
| 2-hexene | cis | 15% |
| | trans | 28% |
| 3-hexene | cis | 7% |
| | trans | 19% |
| | Total | 73% |
| Branched chain dimer | | |
| 2-methyl-1-pentene | | 8% |
| 2-methyl-2-pentene | | 13% |
| 2-methyl-3-pentene | | 5% |
| 2-methyl-4-pentene | | 1% |
| | Total | 27% |

### Example 1

The procedure of Comparative Experiment A was followed, except that an amount of 544 mm 1-butene, 6.3 mm ground palladium cyanide, and as accelerator 6 drops of an aqueous 48% w hydrofluoric acid solution were used. Conversion after 16 h was 16% to a product containing 50 % w dimers.

### Example 2

Dimerization reactions of cis and trans 2-butenes were carried out according to the methods of Comparative Experiment A and Example 1. The resulting dimer selectivities were 50% w. The rate of conversion of cis 2-butene was comparable to that of 1-butene, whilst the conversion rate of trans 2-butene was substantially lower.

### Examples 3, 4 and 5

Three experiments were carried out to investigate the influence of the presence of a small quantity of an acidic accelerating agent upon conversion of propylene and selectivity to dimer. The procedure of Comparative Experiment B was followed, except for the further addition to the autoclave of 1 drop of 50% acetic acid (Example 3), 19% hydrochloric acid (Example 4), or 28% phosphoric acid (Example 5). Conversions were 7, 11, and 15% respectively, and selectivities to dimers 66, 68, and 69% w.

### Claims

1. A process for the selective preparation of dimers of propylene and/or n-butylenes by contacting an olefin reactant selected from the group consisting of propylene, 1-butene, 2-butene, and mixtures thereof, under mechanical agitation with a heterogeneous palladium cyanide catalyst at a temperature in the range from 50°C to 150°C, the quantity of said palladium cyanide being between 0.01 and 10% w calculated on the weight of olefin reactant, characterized in that the contact between the olefin reactant and the catalyst is carried out in the presence of an acid as a reaction accelerator and that said dimers are recovered from the reaction product solution thus obtained.

2. A process as claimed in claim 1, characterized in that the contact between the olefin reactant and the catalyst is carried out in a reaction mixture essentially free of added reaction solvent.

3. A process as claimed in claim 1 or 2, characterized in that the quantity of palladium cyanide is between 0.5 and 4% w, calculated on the weight of olefin reactant.

4. A process as claimed in any one of the preceding claims, characterized in that the accelerator is hydrofluoric acid in a quantity between 5 and 100% w, calculated on palladium cyanide.

5. A process as claimed in any one of the preceding claims, characterized in that the catalyst comprises palladium cyanide on an inert solid support.

6. A process as claimed in claim 5, characterized in that the support is platinum cyanide.

7. A process as claimed in claim 5 or 6, characterized in that the catalyst has been prepared by ball milling the palladium cyanide with the inert support.

**Patentansprüche**

1. Verfahren zur selektiven Herstellung von Dimeren von Propylen und/oder n-Butylenen, bei dem ein Olefin-Reaktionspartner ausgewählt aus der Gruppe bestehend aus Propylen, 1-Buten, 2-Buten und Gemischen daraus unter mechanischem Rühren mit einem heterogenen Palladiumcyanid-Katalysator bei einer Temperatur im Bereich von 50 bis 150°C in Berührung gebracht wird, wobei die Menge des Palladiumcyanids 0,01 bis 10 Gew.-% beträgt, berechnet auf das Gewicht des Olefin-Reaktionspartners, dadurch gekennzeichnet, daß der Kontakt zwischen dem Olefin-Reaktionspartner und dem Katalysator in Gegenwart einer Säure als Reaktionsbeschleuniger vorgenommen wird und daß die Dimeren aus der so erhaltenen Lösung des Reaktionsproduktes gewonnen werden.

2. Verfahren nach Anspruch 1, dadurch gekennzeichnet, daß der Kontakt zwischen dem Olefin-Reaktionspartner und dem Katalysator in einem Reaktionsgemisch vorgenommen wird, das im wesentlichen frei ist von zugesetztem Reaktions-Lösungsmittel.

3. Verfahren nach Anspruch 1 oder 2, dadurch gekennzeichnet, daß die Menge Palladiumcyanid 0,5 bis 4 Gew.-% ausmacht, berechnet auf das Gewicht des Olefin-Reaktionspartners.

4. Verfahren nach einem der vorangehenden Ansprüche, dadurch gekennzeichnet, daß der Beschleuniger Fluorwasserstoffsäure in einer Menge von 5 bis 100 Gew.-% ist, berechnet auf Palladiumcyanid.

5. Verfahren nach einem der vorangehenden Ansprüche, dadurch gekennzeichnet, daß der Katalysator Palladiumcyanid auf einem inerten festen Träger umfaßt.

6. Verfahren nach Anspruch 5, dadurch gekennzeichnet, daß der Träger Platincyanid ist.

7. Verfahren nach Anspruch 5 oder 6, dadurch gekennzeichnet, daß der Katalysator durch Vermahlen des Palladiumcyanids mit dem inerten Träger in einer Kugelmühle hergestellt worden ist.

**Revendications**

1. Procédé de préparation sélective de dimères de propylène et/ou de n-butylènes par mise en contact d'un réactif oléfine choisi dans le groupe constitué par propylène 1-butène, 2-butène, et leurs mélanges, avec agitation mécanique, avec un catalyseur au cyanure de palladium hétérogène à une température située dans un intervalle allant de 50°C à 150°C, la quantité dudit cyanure de palladium étant comprise entre 0,01 et 10% en poids calculée par rapport au poids du réactif oléfine, caractérisé en ce que le contact entre le réactif oléfine et le catalyseur est réalisé en présence d'un acide comme accélérateur de réaction et en ce que lesdits dimères sont recueillis à partir de la solution de produit réactionnel ainsi obtenue.

2. Procédé selon la revendication 1, caractérisé en ce que le contact entre le réactif oléfine et le catalyseur est réalisé dans un mélange réactionnel essentiellement dépourvu de solvant réactionnel ajouté.

3. Procédé selon les revendications 1 et 2, caractérisé en ce que la quantité de cyanure de palladium est comprise entre 0,5 et 4% en poids, calculée par rapport au poids de réactif oléfine.

4. Procédé selon l'une quelconque des revendications précédentes, caractérisé en ce que l'accélérateur est l'acide fluorhydrique en une quantité comprise entre 5 et 100% en poids, calculée par rapport au cyanure de palladium.

5. Procédé selon l'une quelconque des revendications précédentes, caractérisé en ce que le catalyseur comprend du cyanure de palladium sur un support solide inerte.

6. Procédé selon la revendication 5, caractérisé en ce que le support est le cyanure de platine.

7. Procédé selon les revendications 5 et 6, caractérisé en ce que le catalyseur a été préparé en broyant le cyanure de palladium avec le support inerte dans un broyeur à boulet.